(19) Europäisches Patentamt
European Patent Office
Office européen des brevets

(11) Veröffentlichungsnummer: **0 041 674**
**B1**

(12) # EUROPÄISCHE PATENTSCHRIFT

(45) Veröffentlichungstag der Patentschrift: **26.02.86**

(21) Anmeldenummer: **81104169.8**

(22) Anmeldetag: **01.06.81**

(51) Int. Cl.⁴: **C 07 D 263/34,** C 07 D 263/56, C 07 D 413/04, G 03 C 1/727 // G03F7/00

(54) **2-(Trichlormethyl-phenyl)-4-halogen-oxazol-Derivate, ein Verfahren zu ihrer Herstellung und sie enthaltende strahlungsempfindliche Massen.**

(30) Priorität: **09.06.80 DE 3021599**

(43) Veröffentlichungstag der Anmeldung: **16.12.81 Patentblatt 81/50**

(45) Bekanntmachung des Hinweises auf die Patenterteilung: **26.02.86 Patentblatt 86/9**

(84) Benannte Vertragsstaaten: **BE DE FR GB IT NL SE**

(56) Entgegenhaltungen:
**EP - A - 0 010 652**
**DE - A - 2 820 655**
**US - A - 3 912 606**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Dönges, Reinhard, Dr., Oranienstrasse 1, D-6232 Bad Soden (DE)**

**EP 0 041 674 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft 2-(Trichlormethyl-phenyl)-4-halogen-oxazol-Derivate, die in der 5-Stellung des Oxazol-Rings substituiert sind; sie betrifft außerdem ein Verfahren zur Herstellung dieser Verbindungen und diese Verbindungen enthaltende strahlungsempfindliche Massen.

Halogenmethylgruppen aufweisende Verbindungen spielen seit längerer Zeit eine wichtige Rolle als Vor-, Zwischen- und Endprodukte auf zahlreichen Sachgebieten, beispielsweise als Pharmazeutika oder als Bestandteile von strahlungsempfindlichen Massen.

Als Pharmazeutika mit Wirksamkeit gegen Malaria sind beispielsweise Hexachlor-p-xylol

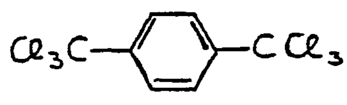

oder 2-(Trichlormethyl-phenyl)-5-trichlormethyl-1.3.4-oxadiazole

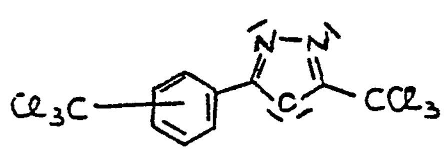

zu nennen (siehe G. Ehrhart und H- Ruschig, mittel", Verlag Chemie - Weinheim, 1972, S. 197/198).

In strahlungsempfindlichen Massen können die Halogenmethylgruppen tragenden Verbindungen als Photoinitiatoren eingesetzt werden, d. h. es werden einerseits die bei Einwirkung von Strahlung aus ihnen entstehenden Radikale zur Auslösung von Polymerisationsreaktionen, Vernetzungsreaktionen oder farblichen Veränderungen ausgenutzt oder es werden andererseits durch die von ihnen freigesetzte Säure Folgereaktionen bewirkt. Zu den seit längerem bekannten Photoinitiatoren zählen beispielsweise Tetrabrommethan $CBr_4$, Tribrom-acetophenon $Br_3C\text{-}CO\text{-}C_6H_5$ und Jodoform $CHJ_3$. Diese relativ leicht zugänglichen Verbindungen absorbieren jedoch nur kurzwelliges UV-Licht, wodurch sie im Anregungsbereich der in der Reproduktionstechnik gebräuchlichen Belichtungslampen nur eine geringe spektrale Empfindlichkeit aufweisen und deshalb mit Hilfe eines zusätzlichen Sensibilisators angeregt werden müssen.

Es wurde versucht, die vorher genannten Schwierigkeiten durch den Einsatz von bestimmte Chromophore aufweisenden halogenorganischen Verbindungen zu überwinden. Aus dem Stand der Technik sind beispielsweise die folgenden Druckschriften bekannt geworden:

Aus der DE-B- 19 49 010 ist die Verwendung von halogenmethylierten Benzophenonen als Initiatoren der Photopolymerisation ungesättigter Verbindungen bekannt, ein Beispiel für einen solchen Photoinitiator ist die folgende Verbindung:

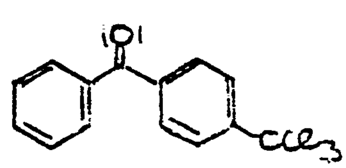

In der DE-C- 20 27 467 wird eine photopolymerisierbare Kopiermasse beschrieben, die eine ethylenisch-ungesättigte polymerisierbare Verbindung, ein Bindemittel und als Photoinitiator eine gegebenenfalls substituierte Verbindung der Acridin- oder Phenazinreihe enthält.

Die s-Triazinderivate der DE-A- 22 43 621 weisen mindestens eine Trihalogenmethylgruppe und mindestens eine chromophore Gruppierung auf, die mit dem Triazinring über ethylenisch-ungesättigte Gruppen ein konjugiertes System bildet; sie sind als Photoinitiatoren in Massen wirksam, die eine ethylenisch-ungesättigte, zur durch Radikale initiierten Additionspolymerisation befähigte Verbindung enthalten. Ein Beispiel für einen solchen Photoinitiator ist die folgende Verbindung:

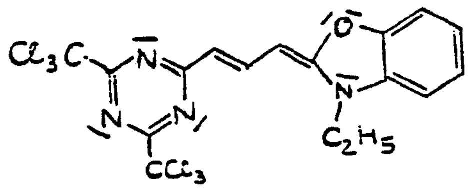

Das durch Belichten löslich werdende Stoffgemisch gemäß der DE-B- 23 06 248 enthält eine wasserunlösliche, durch Einwirkung einer photolytisch gebildeten Säure löslich werdende Verbindung (Aryl-alkyl-acetale und Aryl-alkyl-aminale) und als Photoinitiator eine unter Normalbedingungen neutral reagierende stabile halogenhaltige organische Verbindung, die photolytisch spaltbar ist und dabei eine Säure liefert. Beispiele für geeignete Photoinitiatoren sind: Tetrabromkohlenstoff, Hexabromethan, Trichloracetophenon, Halogenmethyl-s-triazine oder Vinylhalogenmethyl-s-triazine (letztere siehe auch DE-A- 22 43 621).

Aus der DE-A- 27 18 259 ist eine strahlungsempfindliche Masse bekannt, die als strahlungsempfindliche Verbindung ein s-Triazinderivat mit mindestens einer Halogenmethylgruppe und einem zwei- oder mehrkernigen aromatischen Rest als Substituenten enthält; ein Beispiel für eine solche strahlungsempfindliche Verbindung ist die folgende Verbindung:

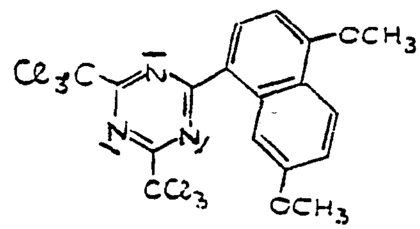

In der DE-A- 28 51 472 wird eine lichtempfindliche Masse beschrieben, die als Photoinitiator ein 2-Halogenmethyl-5-vinyl-1,3,4-oxadiazol-Derivat enthält; ein Beispiel für einen solchen Photoinitiator ist die folgende Verbindung:

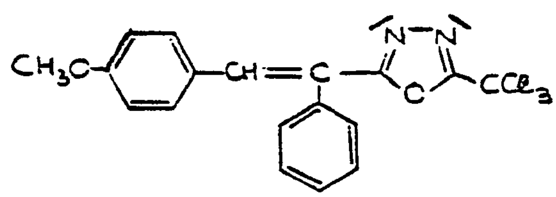

Die Photoinitiatoren der UV-empfindlichen Massen gemäß der US-A 3 912 606 sind Halogenmethylgruppen tragende Benzoxazole, die diese Halogenmethylgruppe direkt oder über einen Benzolring gebunden in der 2-Stellung aufweisen; ein Beispiel für einen solchen Photoinitiator ist die folgende Verbindung:

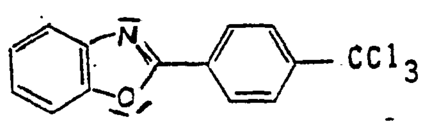

Die aus dem Stand der Technik bekannten Photoinitiatoren bzw. strahlungsempfindlichen Verbindungen weisen allgemein einen oder mehrere der folgenden Nachteile auf:

- die kein photolytisch-spaltbares Halogen aufweisenden Verbindungen können für säurekatalysierte Molekülveränderungen nicht eingesetzt werden (z. B. DE-A-20 27 467),
- bestimmte Synthesen führen in befriedigender Weise nur zu Monohalogenmethyl-substituenten im Molekül (z. B. US-A 3-912 606),
- die Absorptionsmaxima sind zwar gegenüber den einfachen bekannten Photoinitiatoren verschoben, die Absorption ist jedoch immer noch relativ zu kurzwellig (z. B. US-A-3 912 606),
- die Reaktionsbedingungen zur Erzeugung der Verbindungen sind verhältnismäßig drastisch, so daß die Reaktionsausbeute gering ist und die Bildung von unerwünschten Nebenprodukten erleichtert wird (z. B. DE-A-22 43 621, DE-A-27 18 259 oder DE-A-28 51 472), oder
- der Einsatz von bestimmten Katalysatoren erlaubt nur die Anwesenheit von wenigen bestimmten funktionellen Gruppen im Molekül (z. B. DE-A- 27 18 259).

Aus der EP-A-10 652 sind 4-Chlor-1,3-oxazole bekannt, die in 2- und 5-Stellung gegebenenfalls substituierte aromatische Reste tragen. Als Substituenten werden keine Trichlormethylgruppen offenbart. Die Verbindungen sind als optische Aufheller und Photoleiter geeignet.

Aus der OE-A-28 20 655 sind Isoxazole bekannt, die durch Trifluormethylgruppen enthaltende aromatische Reste substituiert sind. Die Verbindungen sind als Mittel zur Regulierung des Pflanzenwachstums geeignet.

Aufgabe der vorliegenden Erfindung ist es deshalb, neue Verbindungen zu synthetisieren, die insbesondere strahlungsempfindlich sind und damit bevorzugt auf dem sich z. Zt. rasch weiterentwickelnden Gebiet der Reproduktionstechnik eingesetzt werden können. Die Verbindungen sollen verhältnismäßig einfach zugänglich sein und eine große Breite an Variationsmöglichkeiten bieten, um für die verschiedensten Bedürfnisse auf dem jeweiligen Anwendungsgebiet optimal angepaßt werden zu können; sie sollen beispielsweise eine spektrale Empfindlichkeit aufweisen, die relativ zur Emission herkömmlicher Strahlungsquellen einen breiten Absorptionsbereich umfaßt, d. h. insbesondere im ultravioletten und kurzwelligen sichtbaren Bereich des Lichtes empfindlich sein. Zusätzlich sollen die Verbindungen, sofern sie in strahlungsempfindlichen

Massen auf dem Reproduktionssektor (beispielsweise auf Druckplatten) eingesetzt werden, bereits nach der Bestrahlung eine deutlich sichtbare farbige Abbildung der Vorlage erzeugen können, wodurch beispielsweise noch vor der eigentlichen Entwicklung der strahlungsempfindlichen Masse Korrekturen von Belichtungsfehlern ermöglicht werden.

Die erfindungsgemäße Lösung dieser Aufgabe sind

2-(Trichlormethyl-phenyl)-4-halogen-oxazol-Derivate der allgemeinen Formel(I)

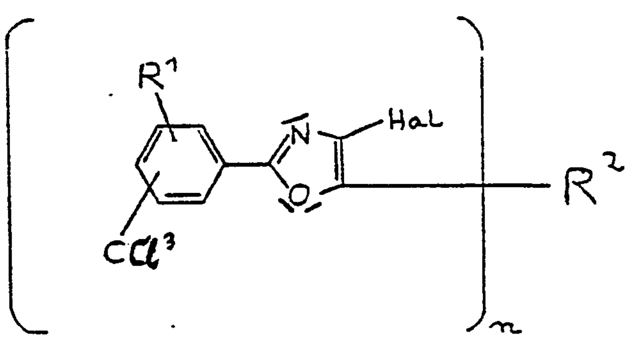

I

in der bedeuten

Hal ein Halogenatom

n die Zahl 1 oder 2

$R^1$ ein Wasserstoffatom oder eine weitere Gruppe $Cl_3$ und

$R^2$ einen n-wertigen, unsubstituierten oder durch Dialkylaminogruppen mit 2 bis 6 Kohlenstoffatomen, Halogenatome, Nitro-, Cyano-, Sulfonyl-, Carboxy-, Trifluormethyl-, Alkyl-, Cycloalkyl-, Alkoxy-, Alkoxycarbonyl-, Carbalkoxyimino-, Carboxyamido-, Phenyl-, Naphthyl-, Indenyl-, Fluorenyl-, Anthryl-, Phenanthryl-, Pyrenyl-, Biphenylyl-, Stilbenyl-, Styryl-,Furyl-, Benzofuryl-, Dibenzofuryl-, Pyrrolyl-, Indolyl-, Carbazolyl-, Thienyl-, Benzothienyl-, Imidazolyl-, Benzimidazolyl-, Oxazolyl-, Benzoxazolyl-, Isoxazolyl-, Thiazolyl-, Benzthiazolyl-, Triazolyl-, Oxdiazolyl-, Thiadiazolyl-, Pyridyl-, Chinolyl-, Isochinolyl-, Acridyl-, Pyrimidyl-, Benzopyranyl-, Benzothianyl- oder Phenoxygruppen substituierten, maximal vierkernigen aromatischen oder heteroaromatischen, gegebenenfalls partiell hydrierten Rest, der an einem ungesättigten Ring-C-Atom direkt oder über eine bis zu vier ausschließlich olefinisch ungesättigten C-Atome enthaltenden Kette mit dem Oxazolyl-Teil des Moleküls gemäß der allgemeinen Formel (I) verbunden ist.

In einer bevorzugten Ausführungsform der Erfindung bedeuten in der allgemeinen Formel (I) Hal ein Chlor- oder Bromatom und n die Zahl 1 oder 2. Besonders bevorzugt werden Verbindungen der allgemeinen Formel I, in denen Hal ein Chloratom, n die Zahl 1, $R^1$ ein Wasserstoffatom und $R^2$ einen durch die oben genannten Substituenten substituierten oder unsubstituierten Phenyl-, Benzofuranyl- oder Naphthylrest bedeutet.

Beispiele für den Rest $R^2$ sind die Reste: Phenyl, Naphthyl, Indenyl, Fluorenyl, Anthryl, Phenanthryl, Pyrenyl, Biphenylyl, Stilbenyl, Styryl, Furyl, Benzofuryl, Dibenzofuryl, Pyrrolyl, Indolyl, Carbazolyl, Thienyl, Benzothienyl, Imidazolyl, Benzimidazolyl, Oxazolyl, Benzoxazolyl, Isoxazolyl, Thiazolyl, Benzthiazolyl, Triazolyl, Oxdiazolyl, Thiadiazolyl, Pyridyl, Chinolyl, Isochinolyl, Acridyl, Pyrimidyl, Benzopyranyl und Benzo-thianyl.

Der Rest $R^2$ kann bevorzugt bis zu vier Substituenten tragen.

Bevorzugte Substituenten am Phenylrest in 2-Stellung des Oxazol-Derivates sind ein Trichlormethylrest in 4- oder 3-Stellung des Phenylrings.

Es ist bekannt, daß sich 4-Halogen-2,5-diaryloxazole durch Einleiten von Halogenwasserstoff bei 0° C in eine Lösung eines Aroylcyanids und eines aromatischen Aldehyds in Ether und anschließende Hydrolyse darstellen lassen [siehe M. Davis, R. Lakhan und B. Ternai, J. Heterocycl. Chem. 14, 317, (1977)].

Die erfindungsgemäßen trichlormethyl-phenylsubstituierten Oxazolderivate lassen sich in vorteilhafter Weise aus einem trichlormethylsubstituierten Benzaldehyd der allgemeinen Formel(II) und einem Carbonsäurecyanid

der allgemeinen Formel (III) herstellen, wobei die folgende Reaktionsgleichunq den Reaktionsverlauf symbolisiert (die Bezeichnungen Hal, n, $R^1$ und $R^2$ haben die weiter oben angegebene Bedeutung):

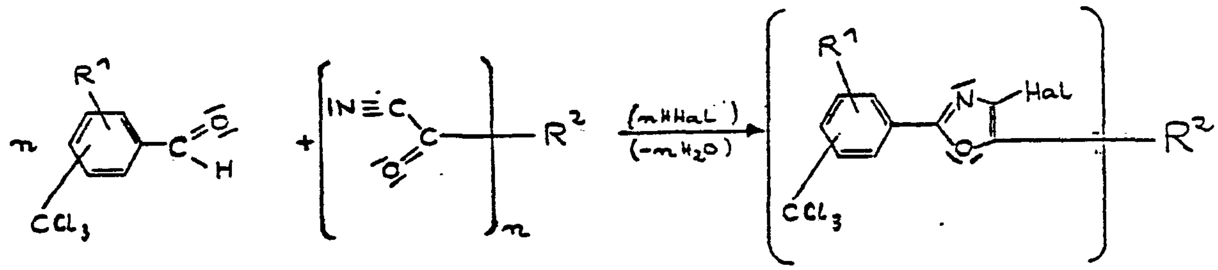


II III I

Die bei dieser unter milden Bedingungen in einem inerten, bevorzugt einen Halogenwasserstoff gut solvatisierenden organischen Lösemittel wie z. B. Tetrahydrofuran, Diethylether, Diisopropylether, Diethylenglykol-diethylether bei Temperaturen zwischen -30°C und +20°C, bevorzugt -20°C bis 0°C, ablaufenden Reaktion einzusetzenden trichlormethylsubstituierten Benzaldehyde sind nur als Monohalogenmethyl-Derivate bekannt (siehe z. B. J. W. Baker, J. A. L. Brieux und D. G. Saunders, J. Chem. Soc. 1956, 404 ff.). Die trichlormethylsubstituierten Benzaldehyde sind neu und mußten erst zugänglich gemacht werden.

Diese neuen trichlormethylsubstituierten Benzaldehyde haben die Formel (II) worin $R^1$ = H oder $CCl_3$ bedeutet, ihre beständigen und gut lagerfähigen Derivate (IV) haben die Formel

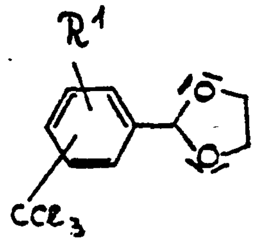

IV

worin $R^1$ die gleiche Bedeutung wie in Formel (II) besitzt. Sie werden hergestellt durch Umsetzung von trichlormethylsubstituierten Benzoesäure-2-methoxy-ethylestern mit einem Oxoniumsalz zu Dioxolaniumsalzen, die ihrerseits dann mit dem komplexen Hydrid eines Blements der III. Gruppe des Periodensystems weiter zu den Verbindungen (IV) umgesetzt werden. Die Verbindungen (II) sind daraus durch Binwirkung wäßriger Säuren erhältlich. Ein weiteres Herstellungsverfahren für die Verbindungen der Formel (II) geht von trichlormethylsubstituierten Benzonitrilen aus, aus denen man durch Umsetzung mit Df-i-butyl-aluminiumhydrid und Zersetzung der dabei gebildeten Al-organischen Komplexverbindungen mit wäßriger Mineralsäure II gewinnt. Schließlich werden die Verbindungen der Formel (II) auch durch Umsetzung von trichlormethylsubstituierten Benzoylhalogeniden mit $NaBH_4$ unter Zusatz eines Metallhalogenids der II. oder III. Nebengruppe wie z. B. CuCl, $CuCl_2$, $ZnCl_2$ oder dem bevorzugt verwendeten $CdCl_2$ in einem inerten polaren Lösungsmittel erhalten.

Die ebenfalls zur Herstellung der erfindungsgemäßen Verbindungen als weitere Komponente benötigten Carbonsäurecyanide lassen sich nach bekannten Verfahren [siehe beispielsweise Organic Sythesis, Coll. Vol. III, 112-114, (1955), DE-OSen 26 42 140, 27 08 182 und 27 08 183, Angew Chem. 68 425 (1956)) herstellen, z. B. durch Umsetzung eines Carbonsäurehalogenids mit einem Metallcyanid wie NaCN, KCN oder CuCN.

Zur Herstellung der erfindungsgemäeen Verbindungen können nahezu alle Carbonsäurecyanide als zweite Komponente neben den Benzaldehyden eingesetzt werden, sofern sie keine säureempfindliche Gruppierung tragen. Besonders geeignet sind Carbonsäurecyanide, die den Chromophor des 2-Phenyloxazols verlängern, so daß die Verbindungen eine sich mit der Emission der gebräuchlichen Belichtungslampen überlagernde Absorption im Bereich von 250 bis 500 nm, bevorzugt 350 bis 400 nm, erhalten. Vorzugsweise werden daher aromatische, olefinische und heterocyclischungesättigte CarbonsäureCyanide eingesetzt, beispielsweise sind folgende Verbindungen geeignet:

Benzoylcyanid, p-Phenylbenzoylcyanid,
3- und 4-Brombenzoylcyanid, p-tert.-Butylbenzoylcyanid,
o-, m- und p-Chlorbenzoylcyanid,
3,4- und 3,5-Dichlorbenzoylcyanid,
4-Dimethylaminobenzoylcyanid, p-Cyanobenzoylcyanid,
2,4- und 2,5-Dimethoxybenzoylcyanid,
3,4- und 3,5-Dimethoxybenzoylcyanid,
3,4,5-Trimethoxybenzcylcyanid,
o-, m- und p-Methoxybenzoylcyanid,
o-, m- und p-Methylbenzoylcyanid,

2,4-, 3,4- und 3,5-Dimethylbenzoylcyanid,
3,5-Dinitrobenzoylcyanid, m- und p-Nitrobenzoylcyanid,
o-, m- und p-Fluorbenzoylcyanid, p-Phenoxybenzoylcyanid,
Naphthalin-1-carbonsäurecyanid,
Naphthalin-2-carbonsäurecyanid,
4-Methylnaphthalin-1-carbonsäurecyanid,
4-Methoxynaphthalin-1-carbonsäurecyanid,
6-Methoxynaphthalin-2-carbonsäurecyanid,
4-Benzoxazolylbenzoylcyanid, Stilben-4-carbonsäurecyanid,
4'-Methoxy-stilben-4-carbonsäurecyanid,
4'-Cyano-stilben-4-carbonsäurecyanid,
Fluoren-2-carbonsäurecyanid,
Terephthaloylcyanid, Isophthalsäuredicyanid,
2,6-Pyridindicarbonsäuredicyanid,
Diphenylether-4,4'-dicarbonsäuredicyanid,
Furancarbonsäurecyanid, Furan-2,5-dicarbonsäuredicyanid,
Benzofurancarbonsäurecyanid,
Cinnamoylcyanid, 4-Chlorcinnamoylcyanid,
3,4-Dimethoxycinnamoylcyanid, o- und p-Methoxycinnamoylcyanid,
3,4,5-Trimethoxycinnamoylcyanid, 4-Nitrocinnamoylcyanid,
4-Methylcinnamoylcyanid.

Die Molverhältnisse der Reaktanten können in weiten Grenzen variiert werden, vorzugsweise wird im Bereich zwischen 0,8 und 1,2 Mol für die eine Komponente pro Mol der anderen Komponente gearbeitet, besonders bevorzugt ist dabei ein ca. 10%iger Überschuß an Carbonsäurecyanid gegenüber dem eingesetzten Aldehyd.

Zu den aus den oben näher beschriebenen Komponenten hergestellten erfindungsgemäßen Verbindungen zählen insbesondere die folgenden, die eine gute Strahlungsempfindlichkeit aufweisen:
2-(p-Trichlormethyl-phenyl)-4-chlor-oxazole, die folgende Substituenten aufweisen:
5-(p-Methoxyphenyl), 5-(p-Chlorphenyl), 5-(4'-Biphenylyl),
5-(p-Benzoxazolylphenyl), 5-(4'-Stilbenyl),
5-(2'-Benzofuranyl), 5-(2'-Naphthyl),
5-(6'-Methoxy-2'-naphthyl) und 5-Styryl.

Die neuen Verbindungen besitzen wegen ihrer Strahlungsempfindlichkeit ein breites Anwendungsspektrum. So sind sie beispielsweise als hochwirksame Starter für Photopolymerisationsreaktionen verwendbar, die durch freie Radikale ausgelöst werden. Geeignete Monomere, die entsprechende Polyadditionen eingehen, sind beispielsweise Mono-, Bis-, Tris- und Tetra-acrylate und -methacrylate von mono- bzw. polyfunktionellen Alkoholen oder Phenolen, Acryl- und Methacrylsäure-amide, abgeleitet von mono- bzw. polyfunktionellen Aminen, Vinylester und Vinylamide. Derartige polymerisierbare Massen können noch zusätzlich Füllstoffe, Bindemittel, Polymerisationsinhibitoren, Farbstoffe, Farbvorläufer, Weichmacher, Haftvermittler oder Sauerstoffänger in unterschiedlichen Mengen beinhalten. Sind diese Massen in Schichtform auf gegebenenfalls chemisch vorbehandelte Träger aufgebracht, d. h. beispielsweise auf Metallfolien aus Stahl, Aluminium, Chrom, Kupfer, Messing, Kunststoff oder Papier, auf Glas, Holz oder Keramik oder auf Verbundmaterialien zweier oder mehrerer dieser Stoffe, kann die lichtempfindliche Schicht auch noch mit einer Deckschicht, die den Zutritt von Sauerstoff hemmt, versehen sein.

Die strahlungsempfindlichen Verbindungen sind als Photoinitiatoren bereits in Konzentrationen von etwa 0,1 % des Gesamtfeststoffs der Masse wirksam, eine Erhöhung über 10 % ist im allgemeinen unzweckmäßig. Vorzugsweise werden Konzentrationen von 1 bis 5 % verwendet.

Weiterhin können die erfindungsgemäßen Verbindungen auch in solchen strahlungsempfindlichen Massen eingesetzt werden, deren Eigenschaftsänderung durch saure Katalysatoren, die bei der Photolyse des Initiators entstehen, eingeleitet wird. Zu nennen sind etwa die kationische Polymerisation von Systemen, die Vinylether, N-Vinylverbindungen wie N-Vinylcarbazol oder spezielle säurelabile Lactone enthalten, wobei nicht ausgeschlossen wird, daß bei einigen dieser Systeme auch radikalische Vorgänge beteiligt sind. Als durch Säuren härtbare Massen sind weiterhin Aminoplaste wie Harnstoff/Formaldehydharze, Melamin/Formaldehydharze und andere N-Methylolverbindungen sowie Phenol/Formaldehydharze zu nennen. Wenn auch die Härtung von Epoxyharzen im allgemeinen durch Lewis-Säuren bzw. solche Säure erfolgt, deren Anionen eine geringere Nukleophilie als Chlorid und Bromid besitzen, also als die Anionen der bei der Photolyse der neuen Verbindungen entstehenden Halogenwasserstoffsäuren, so härten doch Schichten, die aus Epoxyharzen und Novolaken bestehen, in Gegenwart der erfindungsgemäßen Verbindungen glatt aus.

Eine weitere vorteilhafte Eigenschaft der neuen Verbindungen besteht in ihrer Fähigkeit, in gefärbten Systemen bei der Photolyse Farbumschläge hervorzurufen; aus Farbvorläufern, z. B. Leukoverbindungen, Farbbildung zu induzieren oder bathochrome Farbverschiebungen und -vertiefungen in Gemischen zu bewirken, die Cyanin-, Merocyanin- oder Styrylfarbbasen enthalten. Auch kann z. B. in den in der DE-A- 15 72 080 beschriebenen Gemischen, die Farbbase, N-Vinylcarbazol und einen Halogenkohlenwasserstoff enthalten, die Halogenverbindung Tetrabrommethan durch einen Bruchteil ihrer Menge an erfindungs-gemäßer Verbindung ersetzt werden. Farbumschläge sind in der Technik auch z. B. bei der Herstellung von Druckformen erwünscht, um nach der Belichtung bereits vor der Entwicklung das Kopierergebnis beurteilen zu können.

Statt der in den DE-A- 23 31 377 und DE-A- 26 41 100 genannten Säurespender sind vorteilhaft die vorliegenden Verbindungen zu benutzen.

Ein besonders bevorzugtes Anwendungsgebiet für die erfin-dungsgemäßen Verbindungen sind Massen, die neben ihnen als wesentliche Komponente eine Verbindung mit mindestens einer durch Säure spaltbaren C-0-C-Gruppierung enthalten. Als durch Säure spaltbare Verbindungen sind in erster Linie zu nennen:

A) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können und

B) Polymerverbindungen mit wiederkehrenden Acetalund/oder Ketalgruppierungen, bei denen beide $\alpha$-C-Atome der zum Aufbau dieser Gruppierungen erforderlichen Alkohole aliphatisch sind.

Durch Säure spaltbare Verbindungen des Typs A als Komponenten strahlungsempfindlicher Kopiermassen sind in den DE-A- 26 10 842 oder DE-A- 29 28 636 ausführlich beschrieben; Kopiermassen, die Verbindungen des Typs B enthalten, sind Gegenstand der DE-B- 27 18 254.

Als weitere durch Säure spaltbare Verbindungen sind z. B. noch die speziellen Aryl-alkyl-acetale und -aminale der DE-B- 23 06 248 zu nennen, die durch die Photolyseprodukte der erfindungsgemäßen Verbindungen ebenfalls abgebaut werden.

Solche Massen, in denen durch Einwirkung von aktinischer Strahlung mittelbar oder unmittelbar Moleküle, deren Anwesenheit die chemischen und/oder physikalischen Eigenschaften der Masse wesentlich beeinflußt, in kleinere umgewandelt werden, weisen an den bestrahlten Stellen im allgemeinen eine erhöhte Löslichkeit, Klebrigkeit oder Flüchtigkeit auf. Diese Partien können durch geeignete Maßnahmen, beispielsweise Herauslösen mit einer Entwicklungsflüssigkeit, entfernt werden. Bei Kopiermassen spricht man in diesen Fällen von positiv arbeitenden Systemen.

Die bei vielen Positiv-Kopiermassen bewährten Novolak-Kondensationsharze haben sich als Zusatz auch bei der Anwendung der erfindungsgemäßen Verbindungen in Kopiermassen mit durch Säure spaltbaren Verbindungen als besonders brauchbar und vorteilhaft erwiesen. Sie fördern die starke Differenzierung zwischen den belichteten und unbelichteten Schichtpartien beim Entwickeln, besonders die höher kondensierten Harze mit substituierten Phenolen als Formaldehyd-Kondensationspartner. Die Art und Menge der Novolak-Harze kann je nach Anwendungszweck verschieden sein; bevorzugt sind Novolak-Anteile am Gesamtfeststoff zwischen 30 und 90, besonders bevorzugt 55 - 85 Gew.-%.

Zusätzlich können noch zahlreiche andere Harze mitverwendet werden, bevorzugt Vinylpolymerisate wie Polyvinylacetate, Polyacrylate, Polyvinylether und Polyvinylpyrrolidone, die selbst durch Comonomere modifiziert sein können. Der günstigste Anteil an diesen Harzen richtet sich nach den anwendungstechnischen Erfordernissen und dem Einfluß auf die Entwicklungsbedingungen und beträgt im allgemeinen nicht mehr als 20 % vom Novolak. In geringen Mengen kann die lichtempfindliche Masse für spezielle Erfordernisse wie Flexibilität, Haftung und Glanz etc. außerdem noch Substanzen wie Polyglykole, Cellulose

Derivate wie Ethylcellulose, Netzmittel, Farbstoffe undfeinteilige Pigmente sowie bei Bedarf UV-Absorber enthalten. Entwickelt wird vorzugsweise mit in der Technik üblichen wäßrig-alkalischenn Entwicklern, die auch kleine Anteile organischer Lösemittel enthalten können.

Die bereits im Zusammenhang mit den photopolymerisierbaren Massen aufgeführten Träger kommen ebenfalls für positiv arbeitende Kopiermassen in Frage, zusätzlich die in der Mikroelektronik üblichen Silizium- und Siliziumdioxidoberflächen.

Die Menge der als Photoinitiator eingesetzten erfin-dungsgemäßen Verbindungen kann in den positiv arbeitenden Kopiermassen je nach Substanz und Schicht sehr verschieden sein. Günstigere Ergebnisse werden erhalten zwischen etwa 0,1 und 10 %, bezogen auf den Gesamtfeststoff, bevorzugt sind etwa 1 bis 5 %. Für Schichten mit Dicken über 10 $\mu$m empfiehlt es sich, relativ wenig Säurespender zu verwenden.

Grundsätzlich ist elektromagnetische Strahlung mit Wellenlängen bis etwa 600 nm geeignet, in den die erfindungsgemäße Verbindung enthaltenden strahlungsempfindlichen Massen Reaktionen der beschriebenen Art auszulösen. Der bevorzugte Wellenlängenbereich erstreckt sich von 250 bis 500 nm.

Die Vielfalt der erfindungsgemäßen Verbindungen, deren Absorptionsmaxima teilweise noch weit im sichtbaren Teil des Spektrums zu finden sind und deren Absorptionsbereich über 500 nm hinausreichen kann, gestattet es, den Photoinitiator optimal auf die verwendete Lichtquelle abzustimmen. Prinzipiell ist jedoch auch eine Sensibilisierung nicht ausgeschlossen. Als Lichtquellen sind beispielsweise zu nennen:

Röhrenlampen, Xenonimpulslampen, metallhalogendotierte Quecksilberdampf-Hochdrucklampen und Kohlebogenlampen.

Darüber hinaus ist bei den erfindungsgemäßen lichtempfindlichen Kopiermassen das Belichten in üblichen Projektions- und Vergrößerungs-Geräten unter dem Licht der Metallfadenlampen und Kontakt-Belichtung mit gewöhnlichen Glühbirnen möglich. Die Belichtung kann auch mit kohärentem Licht eines Lasers erfolgen. Geeignet für die Zwecke vorliegender Erfindung sind leistungsgerechte kurzwellige Laser, beispielsweise Argon-Laser, Kryptonlonen-Laser, Farbstoff-Laser und Helium-Cadmium-Laser, die insbesondere zwischen 250 und 500 nm emittieren. Der Laserstrahl wird mittels einer vorgegebenen programmierten Strich- und/oder Raster-Bewegung gesteuert.

Das Bestrahlen mit Elektronenstrahlen ist eine weitere Differenzierungsmöglichkeit. Elektronenstrahlen können Kopiermassen, die eine der erfindungsgemäßen Verbindungen und eine durch Säure spaltbare Verbindung enthalten, wie auch viele andere organische Materialien durchgreifend zersetzen und vernetzen, so daß ein negatives Bild entsteht, wenn die unbestrahlten Teile durch Lösemittel oder Belichten ohne Vorlage und

Entwickeln entfernt werden.

Bei geringerer Intensität und/oder höherer Schreibgeschwindigkeit des Elektronenstrahls bewirkt dagegen der Elektronenstrahl eine Differenzierung in Richtung höherer Löslichkeit, d. h. die bestrahlten Schichtpartien können vom Entwickler entfernt werden. Die günstigsten Bedingungen können durch Vorversuche leicht ermittelt werden.

Bevorzugte Anwendung finden die eine der erfindungsge-mäßen Verbindungen enthaltenden strahlungsempfindlichen Massen bei der Herstellung von Druckformen, d. h. insbesondere Offset-, autotypischen Tiefdruck- und Siebdruckformen, in Kopierlacken und in sogenannten Trockenresists.

Die folgenden Beispiele dienen zur näheren Erläuterung der Erfindung, es erfolgt zunächst die Beschreibung von verschiedenen erfindungsgemäßen Verbindungen, woran sich die Anwendung von einigen dieser Verbindungen in strahlungsempfindlichen Massen anschließt.

In den Beispielen stehen Gew.-Teile (Gt) und Vo1.-Teile (Vt) im Verhältnis von g zu ml. Prozent- und Mengenangaben sind, wenn nichts anderes angegeben ist, in Gewichtseinheiten zu verstehen.

**Tabelle 1**

Beispiele für Verbindungen der allgemeinen Formel (VI)

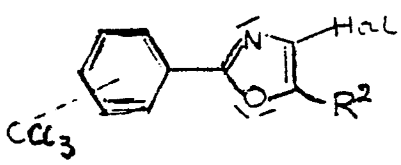

VI

(d. h. Formel 1 mit R = H und n = 1),
wobei die Verbindungen 1 bis 14 die Trichlormethylgruppe in 4-Stellung und die Verbindung 15 die Trichlormethylgruppe in 3-Stellung des Phenylrings aufweisen; abweichend davon ist bei Verbindung 14 der Rest $R^2$ zweibindig und damit wird n = 2.

| Verbindung Nr. | $R^2$ = | Hal = | Schmp. (°C) | langwelliges Absorptions-maximum in DMF $\lambda_{max}$ (lgε) [nm] |
|---|---|---|---|---|
| 1 | 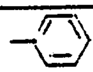 | Cl | 129-30 | 326 (4,40) |
| 2 | 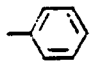 | Br | 123-25 | 325 (4,40) |
| 3 | 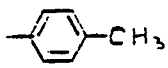 | Cl | 150-51 | 332 (4,41) |
| 4 | 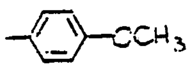 | Cl | 170-71 | 340 (4,40) |
| 5 | 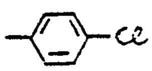 | Cl | 154-55 | 326 (4,46) |
| 6 | 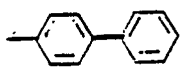 | Cl | 152 | 343 (4,56) |
| 7 | 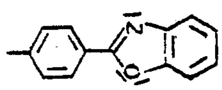 | Cl | 259-60 | 359 (4,71) |
| 8 | 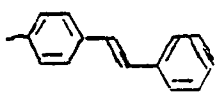 | Cl | 160-62 | 366 (4,71) |

| Verbindung Nr. | $R^2$ = | Hal = | Schmp. (°C) | langwelliges Absorptions-maximum in DMF $\lambda_{max}$ (lg ε) [nm] |
|---|---|---|---|---|
| ~~11~~ 9 | 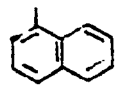 | Cl | 121-22 | 323 (4,33) |
| ~~12~~ 10 | 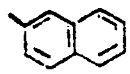 | Cl | 162-64 | 341 (4,49) |
| ~~13~~ 11 | 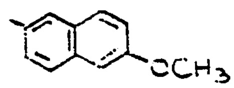 | Cl | 209-11 | 351 (4,48) |
| ~~14~~ 12 | 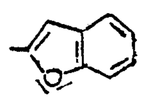 | Cl | 176-79 | 349 (4,48) |
| ~~15~~ 13 | 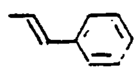 | Cl | 139-40 | 359 (4,50) |
| ~~16~~ 14 | 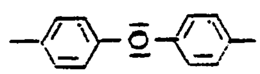 | Cl | 222-24 | 342 (4,74) |
| ~~17~~ 15 | 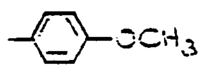 | Cl | 161-62 | 330 (4,37) |

**Beispiel 1** (Verbindungen 4)

Eine Lösung von 8 g des p-Trichlormethylbenzaldehyds und 6,5 g des p-Methoxybenzoylcyanids in 40 ml trockenem Tetrahydrofuran wird bei -20° bis -30° C mit Chlorwasserstoffgas gesättigt. Nach 15 Stunden bei 0° C wird auf Eis gegossen. Das ausgefallene Produkt wird abgesaugt und aus Aceton umkristallisiert.

Ausbeute 11,2 g (77 % d. Th.)
$C_{17}H_{11}Cl_4NO_2$ ber: C 50,66, H 2,75 N 3,47 Cl 35,18
[403,09] gef: C 50,7 H 2,7 N 3,5 Cl 35,1
Schmp: 170 - 171° C
$^1$H-NMR-Speütrum ($CDCl_3$):δ = 3,85 (s, OCH), 7,0 (d, J = 9 Hz, 2 H), 7,85 (d, J = 9 Hz, 2 H), 8,05 (s, 4 H)
UV-Spektrum (DMF):$\lambda_{max}$ (ε) = 340 nm (25100)

**Beispiel 2** (Verbindung 5)

11,5 g eines zu 70 % aus p-Trichlormethylbenzaldehyd und zu 30 % aus p-Trichlormethylbenzonitril bestehenden Gemisches und 6,5 g des p-Chlorbenzoylcyanids werden in 40 ml trockenem Tetrahydofuran nach den Angaben des Beispiels 1 umgesetzt. Nach dem Umkristallisieren aus Aceton werden 11,6 g (79 % d. Th.) der Verbindung 5 erhalten.

$C_{16}H_8Cl_5NO$ ber: C 47,16 H 1,98 N 3,44 Cl 43,50 [407,51] gef: C 47,2 H 2,0 N 3,5 Cl 43,2
Schmp: 154 - 155° C
$^1$H-NMR-Spektrum (CDCl): δ = 7,4 (d, J = 9 Hz, 2 H), 7,85 (d, J.= 9 Hz, 2 H), 8,05 (s, 4 H)
UV-Spektrum (DMF): $\lambda_{max}$ (ε) = 326 (28800), 332 nm (S, 28600)

**Beispiel 3** (Verbindung 6)

8 g des Trichlormethylbenzaldehyds und 8,3 g des p-Phenylbenzoylcyanids werden nach den Angaben des Beispiels 1 umgesetzt.
Ausbeute 11,1 g (67 % d. Th.)
$C_{22}H_{13}Cl_4NO$ ber:C58,83 H 2,92 N 3,12 Cl 31,57
[449,16] gef:C58,7 H 3,2 N 3,1 Cl 31,5
Schmp: 152°C
$^1$H-NMR-Spektrum ($CDCl_3$): $\delta$ = 7,3 - 7,7 (m, 5 H), 7,7 (d, J = 9 Hz, 2 H), 8,0 (d, J = 9 Hz, 2 H), 8,05 (s, 4 H)
UV-Spektrum (DMF):$\lambda_{max}$ ($\varepsilon$) = 277 (17700), 343 nm (36000)

**Beispiel 4** (Verbindung 13)

4,2 g des Cinnamoylcyanids werden nach den Angaben des Beispiels 1 umgesetzt.
Ausbeute 11,4 g (79 % d. Th.)
$C_{18}H_{11}Cl_4NO$ ber: C 54,17 H 2,78 N 3,51 Cl 35,53
[399,10] gef: C 54,3 H 2,8 N 3,5 Cl 34,4
Schmp: 139 - 140°C
$^1$H-NMR-Spektrum ($CDCl_3$): $\delta$ = 6,85 (d, J = 16 Hz, olef. H), 7,2 (d, J = 16 Hz, olef. H), 7,4 (m, 5 H), 8,05 (s, 4 H)
UV-Spektrum (DMF):$\lambda_{max}$ ($\varepsilon$) = 359 nm (31800)

**Beispiel 5** (Verbindung 14)

15 g eines zu 70 % aus p-Trichlormethylbenzaldehyd und zu 30 % aus p-Trichlormethylbenzonitril bestehenden Gemisches werden mit 5,8 g des 4,4'-Bis-(cyanocarbonyl)-diphenylether nach den Angaben des Beispiels 2 umgesetzt. Nach dem Umkristallisieren aus Essigester werden 6,3 g (41 % d. Th.) der Verbindung 14 erhalten.
$C_{32}H_{16}Cl_8N_2O_3$ ber: C 50,56 H 2,12 N 3,69 Cl 37,31
[760,12] gef: C 51,0 H 2,2 N 3,6 Cl 37,0
Schmp: 222 - 224°C
$^1$H-NMR-Spektrum ($CDCl_3$): $\delta$= 7,15 (d, J = 9 Hz, 4 H), 7,95 (d, J = 9 Hz, 4 H), 8,05 (s, 8 H)
UV-Spektrum (DMF):$\lambda_{max}$ ($\varepsilon$) = 342 nm (55000)

**Beispiel 6** (Verbindung 15)

8 g des Trichlormethylbenzaldehyds werden nach den Angaben des Beispiels 1 umgesetzt.
Ausbeute 12,3 g (85 % d. Th.)
$C_{17}H_{11}Cl_4NO_2$ ber: C 50,66 H 2,75 N 3,47 Cl 35,18
[403,09] gef: C 50,4 H 2,9 N 3,5 Cl 35,2
Schmp: 161 - 162°C
$^1$H-NMR-Spektrum ($CDCl_3$):$\delta$ = 3,85 (s, OCH), 7,0 (d, J = 9 Hz, 2 H), 7,5 (t, J = 8 Hz, H), 7,85 (d, J = 9 Hz, 2 H), 8,05 (m, 2 H), 8,6 (breites s, H)
UV-Spektrum (DMF):$\lambda_{max}$ ($\varepsilon$) = 330 nm (23300)

**Beispiel 7**

Eine elektrochemisch aufgerauhte Aluminiumplatte wird mit einer Lösung bestehend aus:
6,5 Gt Trimethylolethantriacrylat
6,5 ' Methacrylsäure/Methylmethacrylat-Copolymerem (Säurezahl 115)
0,2 ' Verbindung (Photoinitiator) 1
64,0 ' Ethylenglykolmonoethylether
22,6 ' Butylacetat
0,2 ' 2,4-Dinitro-6-chlor-2'-acetamido-5'-methoxy-4'-(ß-hydroxyethyl-ß'-cyanoethyl)amino-azobenzol
beschichtet, so daß sich ein Schichtgewicht von etwa 3 bis 4 g/m$^2$ nach dem Trocknen ergibt. Die so beschichtete Platte wird zusätzlich mit einem Polyvinylalkoholdeckstrich von 4 µm Dicke versehen und in einem Vakuumbelichtungsrahmen mit Leuchtröhren TLAK 20W/05 der Firma Philips aus einem Abstand von ca. 10 cm kontaktbelichtet. Das negative Bild der Vorlage wird mit einer 1,5%igen wäßrigen Natriummetasilikat-Lösung

entwickelt.

In Tabelle II ist die Anzahl der ausgehärteten Stufen eines Stufenkeils aufgeführt, wobei der Photoinitiator 1 durch eine äquimolare Menge eines anderen Photoinitiators ersetzt wird. Eine Differenz von zwei Keilstufen entspricht dabei der doppelten Lichtempfindlichkeit der lichtempfindlichen Schicht.

**Tabelle II**

| Verbindung Nr. | ausgehärtete Keilstufen nach Beispiel 1 (t = Belichtungszeit) | |
|---|---|---|
| | t = 45 sec | t = 2 min |
| 1 | 6 | 10 |
| 2 | 5 | 9 |
| 3 | 7 | 9 |
| 4 | 7 | 10 |
| 5 | 7 | 10 |
| 6 | 7 | 10 |
| 7 | 4 | 7 |
| 8 | 9 | 11 |
| ~~9~~ | ~~6~~ | ~~9~~ |
| ~~10~~ | ~~5~~ | ~~9~~ |
| ~~11~~ 9 | 4 | 7 |
| ~~12~~ 10 | 7 | 9 |
| ~~13~~ 11 | 8 | 11 |
| ~~14~~ 12 | 8 | 10 |
| ~~15~~ 13 | 8 | 10 |
| ~~16~~ 14 | 5 | 9 |
| ~~17~~ 15 | 4 | 7 |

**TABELLE II**

| Verbindung Nr. | ausgehärtete Keilstufen nach Beispiel 1 (t = Belichtungszeit) | |
|---|---|---|
| | t = 45 s | t = 2 min |
| Bis(trichlormethyl)-(4-methoxystyryl)-s-triazin nach DE-A- 22 43 621 | 5 | 8 |
| 9-Phenylacridin nach DE-C- 20 27 467 | 10 | 13 |

Die in der Praxis eingesetzte, ebenfalls eine Trihalogenmethylgruppe aufweisende Verbindung gemäß der DE-A- 22 43 621 wird von einer großen Anzahl an erfindungsgemäßen Produkten in der Wirksamkeit übertroffen. Die ebenfalls in der Praxis eingesetzte, keine Trihalogenmethylgruppe aufweisende Verbindung gemäß der DE-A- 20 27 467 ist zwar in der vorliegenden lichtempfindlichen Masse etwas wirksamer als die hier aufgeführten Beispiele der erfindungsgemäßen Verbindungen, sie kann jedoch nur in strahlungsempfindlichen Massen eingesetzt werden, die photopolymerisierbare Monomere enthält, da das photolytisch-abspaltbare Halogen fehlt; d. h. ein Einsatz in strahlungsempfindlichen Massen, die eine Verbindung enthalten, deren Löslichkeit durch Einwirkung von Säure verändert wird, kann - im Gegensatz zu den erfindungsgemäßen Verbindungen - dann nicht erfolgen.

**Beispiel 8**

Eine Schichtzusammensetzung gemäß Beispiel 8 unter Verwendung von 0,2 Gt der lichtempfindlichen Verbindunq 1 wird vor der Belichtung einem Lagertest bei 100°C unterworfen. Wie Tabelle III zeigt, läßt sich kein Aktivitätsverlust feststellen.

**Tabelle III**

| Lagerzeit [min] bei 100°C vor der Belichtung | ausgehärtete Keilstufen unter Verwendung des Initiators 1 (t = 2 min) |
|---|---|
| 0 | 9 |
| 60 | 8 |
| 90 | 8 |
| 120 | 7 |
| 150 | 7 |
| 180 | 7 |
| 210 | 7 |
| 240 | 8 |

der Belichtung

**Beispiel 9**

Eine elektrochemisch aufgerauhte Aluminiumplatte wird mit einer Lösung bestehend aus
6,5 Gt Trimethylolethantriacrylat
6,5 ' Methacrylsäure/Methylmethacrylat-Copolymerem (Säurezahl 115)
0,2 ' Photoinitiator 4
64,0 ' Ethylenglykolmonoethylether
22,6 Gt Butylacetat
0,24 ' Leukomalachitgrün
beschichtet und mit einem Polyvinylalkohol-Deckstrich versehen. Nach einer Belichtungszeit von 60 sec gemäß Beispiel 7 wird ein negatives, grünes Bild der Vorlage erhalten, das durch Entwicklung zur druckfähigen Platte fixiert wird. Wird Leukomalachitgrün durch Kresolrot ersetzt, so erhält man ein rotes Bild der Vorlage.

**Beispiel 10**

Ein gebürstetes Aluminiumblech wird im Tauchverfahren mit einer Lösung bestehend aus
10 Vt Methylethylketon
1 Gt Kresol-Formaldehyd-Novolak
0,3 ' Triethylenglykol-Ethylbutyraldehyd-Polykondensat
0,015 ' einer der erfindungsgemäßen Verbindungen
beschichtet, gemäß Beispiel 7 belichtet und das positive Bild der Vorlage mit einer
5,5 Gt Natriummetasilikat - 9 $H_20$
3,4 ' Trinatriumphosphat - 12 $H_20$
0,4 ' Natriumdihydrogenphosphat
90,7 ' entsalztes Wasser
enthaltenden Lösung entwickelt. In Tabelle IV ist die Anzahl der entwickelbaren Keilstufen angegeben.

**TABELLE IV**

| Verbindung Nr. | Keilstufen (t = 2 min) | Verbindung Nr. | Keilstufen (t = 2 min) |
|---|---|---|---|
| 1 | 7 | ~~12~~ 10 | 10 |
| 2 | 6 | ~~13~~ 11 | 11 |
| 3 | 8 | ~~14~~ 12 | 10 |
| 4 | 11 | ~~15~~ 13 | 11 |
| 5 | 7 | ~~16~~ 14 | 10 |
| 6 | 8 | ~~17~~ 15 | 5 |
| 7 | 8 | Bis(trichlor- | |
| 8 | 11 | methyl)-(4- | |
| ~~9~~ | ~~7~~ | methoxystyryl)- | |
| ~~10~~ | ~~6~~ | s-triazin nach | |
| ~~11~~ 9 | 7 | DE-~~OS~~ A- 22 43 621 | 9 |

Die in der Praxis eingesetzte Verbindung gemäß der DE-A— 22 43 621 wird von vielen der erfindungsgemäßen Verbindungen in der Wirksamkeit übertroffen.

**Beispiel 11**

Einer positiv abbildenden Schicht gemäß Beispiel 10 werden 5 mg Methylrot oder Kresolrot zugemischt. Nach der Belichtung wird ein rot-gefärbtes Bild der Vorlage erhalten.

**Beispiel 12**

Gemäß Beispiel 7 wird eine Schicht unter Verwendung des Photoinitiators 12 hergestellt und unterschiedlichen Belichtungszeiten unterworfen. Die Anzahl der jeweils ausgehärteten Keilstufen steigt linear mit der BelichtungsdaVer (Tabelle V).

**Tabelle V**

| Belichtungsdauer [sec] | ausgehärtete Keilstufen |
|---|---|
| 10 | 1 |
| 20 | 3 |
| 40 | 6 |
| 80 | 8 |
| 160 | 10 |
| 320 | 12 |

**Patentansprüche**

1. 2-(Trichlormethyl-phenyl)-4-halogen-oxazol-Derivate der allgemeinen Formel (I)

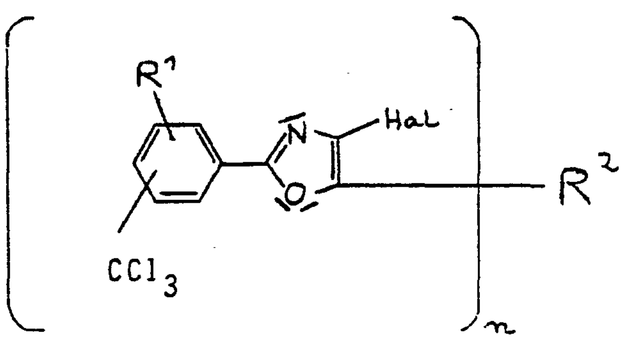

I

in der bedeuten
Hal ein Halogenatom,
n die Zahl 1 oder 2,
$R^1$ ein Wasserstoffatom oder eine weitere Gruppe $CCl_3$ und
$R^2$ einen n-wertigen, unsubstituierten oder durch Dialkylaminogruppen mit 2 bis 6 Kohlenstoffatomen, Halogenatome, Nitro-, Cyano-, Sulfonyl-, Carboxy-, Trifluormethyl-, Alkyl-, Cycloalkyl-, Alkoxy-, Alkoxycarbonyl-, Carbalkoxyimino-, Carboxyamido-, Phenyl-, Naphthyl-, Indenyl-, Fluorenyl-, Anthryl-, Phenanthryl-, Pyrenyl-, Biphenylyl-, Stilbenyl-, Styryl-, Furyl-, Benzofuryl-, Dibenzofuryl-, Pyrrolyl-, Indolyl-, Carbazolyl-, Thienyl-, Benzothienyl-, Imidazolyl-, Benzimidazolyl-, Oxazolyl-, Benzoxazolyl-, Isoxazolyl-, Thiazolyl-, Benzthiazolyl-, Triazolyl-, Oxdiazolyl-, Thiadiazolyl-, Pyridyl-, Chinolyl-, Isochinolyl-, Acridyl-, Pyrimidyl-, Benzopyranyl-, Benzothianyl- oder Phenoxygruppen substituierten, maximal vierkernigen aromatischen oder heteroaromatischen, gegebenenfalls partiell hydrierten Rest, der an einem ungesättigten Ring-C-Atom direkt oder über eine bis zu vier ausschließlich olefinisch ungesättigten C-Atome enthaltenden Kette mit dem Oxazolyl-Teil des Moleküls gemäß der allgemeinen Formel (I) verbunden ist.

2. Verbindungen der allgemeinen Formel (I) nach Anspruch 1, in der
n die Zahl 1 oder 2 und
Hal ein Chlor- oder Bromatom
bedeuten.

3. Verbindungen der allgemeinen Formel

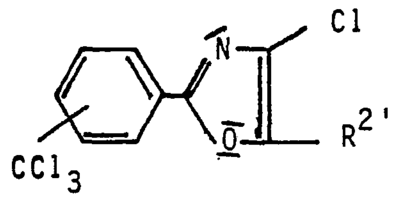

in der

$R^{2'}$ einen gemäß Anspruch 1 substituierten oder unsubstituierten Phenyl-, Benzofuranyl- oder Naphthylrest bedeutet.

4. Verwendung einer Verbindung nach einem der Ansprüche 1 bis 3 als strahlungsempfindliche Verbindung in einer strahlungsempfindlichen Masse.

5. Strahlungsempfindliche Masse, die a) als strahlungsempfindliche Verbindung 0,1 bis 10 Gew.-% einer Verbindung nach einem der Ansprüche 1 bis 3 und b) eine ethylenisch ungesättigte Verbindung, die eine durch freie Radikale ausgelöste Polymerisation eingehen kann, eine Verbindung, deren Löslichkeit durch Einwirkung von Säure verändert wird, oder eine Verbindung enthält, die durch saure Katalysatoren zur kationischen Polymerisation veranlaßt wird.

6. Strahlungsempfindliche Masse nach Anspruch 5, dadurch gekennzeichnet, daß sie als Verbindung, deren Löslichkeit durch Einwirkung von Säuren erhöht wird, eine Verbindung mit mindestens einer durch Säure spaltbaren C-0-C-Gruppierung enthält.

7. Verfahren zur Herstellung der Verbindungen nach Anspruch 1, dadurch gekennzeichnet, daß man einen Trichlormethyl-benzaldehyd der allgemeinen Formel (II) mit einem Carbonsäurecyanid der allgemeinen Formel (III)

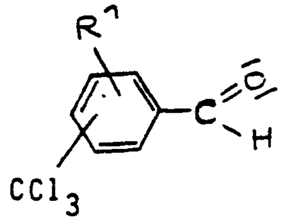

II

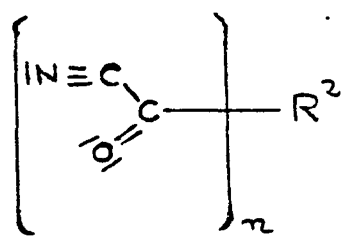

III

unter Einwirkung von HHal umsetzt und die Reaktionsprodukte durch Hydrolyse freisetzt, wobei die Bezeichnungen Hal, n, $R^1$ und $R^2$ die in Anspruch 1 angegebene Bedeutung haben.

## Claims

1. 2-(trichloromethyl-phenyl)-4-halogeno-oxazole derivatives of the general formula 1

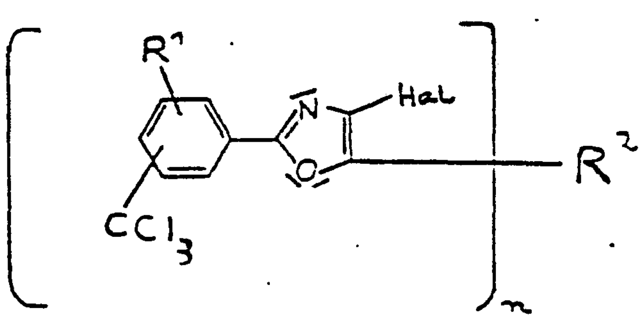

I

wherein:

Hal represents a halogen atom,

n is 1 or 2,

$R^1$ represents a hydrogen atom or a further $CCl_3$ group, and

$R^2$ represents a n-valent, at most tetranuclear aromatic or heteroaromatic, optionally partially hydrogenated radical, which is linked, at an unsaturated ring carbon atom, directly or by means of a chain containing up to 4 exclusively olefinically unsaturated carbon atoms, to the oxazolyl moiety of the molecule of the general formula

(I), which radical is unsubstituted or substituted by dialkylamino groups having from 2 to 6 carbon atoms, halogen atoms, nitro, cyano, sulfonyl, carboxyl, trifluoromethyl, alkyl, cycloalkyl, alkoxy, alkoxycarbonyl, carbalkoxyimino, carboxyamido, phenyl, naphthyl, indenyl, fluorenyl, anthryl, phenanthryl, pyrenyl, biphenylyl, stilbenyl, styryl, furyl, benzofuryl, dibenzofuryl, pyrrolyl, indolyl, carbazolyl, thienyl, benzothienyl, imidazolyl, benzimidazolyl, oxazolyl, benzoxazolyl, isoxazolyl, thiazolyl, benzthiazolyl, triazolyl, oxadiazolyl, thiadiazolyl, pyridyl, quinolyl, isoquinolyl, acridyl, pyrimidyl, benzopyranyl, benzothianyl or phenoxy groups.

2. Compounds of the general formula I, as claimed in claim 1, wherein:
Hal represents a chlorine or bromine atom, and
n is the number 1 or 2.

3. Compounds of the general formula

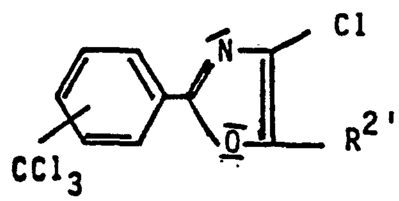

wherein:
$R^{2'}$ represents a phenyl, benzofuranyl or naphthyl radical substituted according to claim 1 or unsubstituted.

4. The use of a compound as claimed in any of claims 1 to 3, as a radiation-sensitive compound in a radiation-sensitive composition.

5. A radiation-sensitive composition which, as the radiation-sensitive compound, contains
(a) from 0.1 to 10 % by weight of a compound as claimed in any of claims 1 to 3 and
b) an ethylenically unsaturated compound which is capable of undergoing a polymerization reaction initiated by free radicals, a compound, the solubility of which is modified by the action of an acid, or a compound which is caused by acid catalysts to undergo a cationic polymerization.

6. A radiation-sensitive composition as claimed in claim 5, which comprises as the compound the solubility of which is increased by the action of acids a compound which has at least one C-0-C grouping which can be split by acid.

7. A process for the preparation of the compounds as claimed in claim 1, which comprises reacting a trichloromethyl-benzaldehyde of the general formula II with a carboxyclic acid cyanide of the general formula III

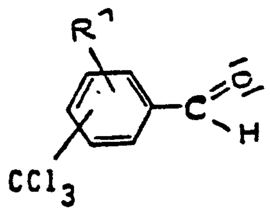

II

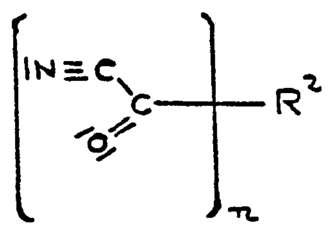

III

under the action of HHal and liberating the reaction product by hydrolysis, the symbols Hal, n, $R^1$ and $R^2$ having the meaning indicated in claim 1.

## Revendications

1 - Dérivés de 2-(trichloromethyl-phenyl)-4-halo-géno-oxazole, répondant à la formule générale (I)

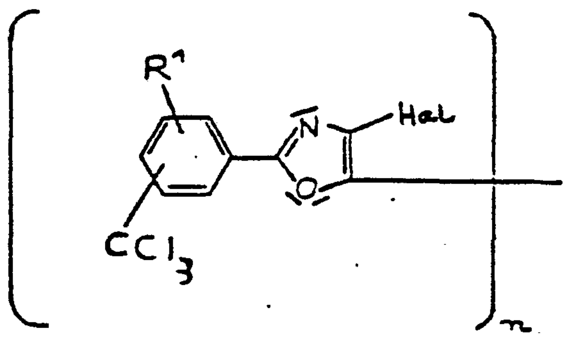

I

dans laquelle
Hal représente un atome d'halogéne,
n est le nombre 1 ou 2,
$R^1$ représente un atome d'hydrogene ou un autre groupe CCl et
$R^2$ représente un radical aromatique ou hétéro-aromatique, de 4 noyaux au maximum, éventuellement partiellement hydrogéné, de valence n, non-substitué ou substitué par des groupes dialcoylamino ayant de 2 à 6 atomes de carbone, des atomes d'halogène, des groupes nitro, cyano, sulfonyle, carboxy, trifluorométhyle, alcoyle, cycloalcoyle, alkoxy, alkoxycarbonyle, carbalkoxyimino, carboxyamido, phényle, naphtyle, indényle, fluorényle, anthryle, phénanthryle, pyrényle, biphénylyle, stilbényle, styryle, furyle, benzofuryle, dibenzofuryle, pyrrolyle, indolyle, carbazolyle, thiényle, benzothienyle, imidazolyle, benzimidazolyle, oxazolyle, benzoxazolyle, isoxazolyle, thiazolyle, benzothiazolyle, triazolyle, oxodiazolyle, thiadiazolyle, pyridyle, quinolyle, isoquinolyle, acridyle, pyrimidyle, benzopyrannyle, benzothiannyle ou phénoxy, lequel radical est relié sur un atome de carbone cyclique insaturé, directement ou par l'intermédiaire d'une chaîne contenant jusqu'à 4 atomes de carbone à insaturation exclusivement oléfinique, avec la partie oxazolyle de la molécule selon la formule générale (I).

2 - Composés de formule générale (I) selon la revendication 1, dans lesquels:
n représente le nombre 1 ou 2 et
Hal représente un atome de chlore ou de brome.

3 - Composés de formule générale

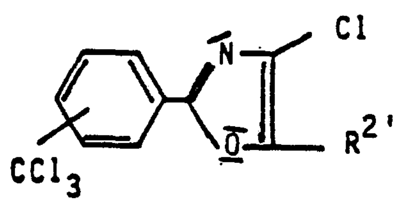

dans laquelle:
$R^2$ représente un radical phènyle, benzofurannyle ou naphtyle, substitué selon la revendication 1 ou non-substitue.

4 - Utilisation d'un composé selon l'une quelconque des revendications 1 à 3 comme composé sensible aux radiations dans une composition sensible aux radiations.

5 - Composition sensible aux radiations qui contient a) en tant que composé sensible aux radiations de 0,1 à 10 % en poids d'un composé selon l'une quelconque des revendications 1 à 3, et b) un composé à insaturation éthylénique qui peut introduire une polymérisation initiée par des radicaux libres, un composé dont la solubilité est modifiée sous l'influence d'un acide, ou un composé qui donne lieu à une polymérisation cationique par l'intermédiaire de catalyseurs acides.

6 - Composition sensible aux radiations selon la revendication 5, caractérisée en ce qu'elle contient, comme composé dont la solubilité est augmentée sous l'influence des acides, un composé avec au moins un groupe C-O-C séparable par un acide.

7 - Procédé pour la préparation descomposés selon la revendication 1, caractérisé en ce qu'on fait réagir un trichlorométhyl-benzaldéhyde de formule générale (II) avec un cyanure d'acide carboxylique de formule générale (III)

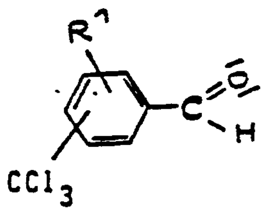 II

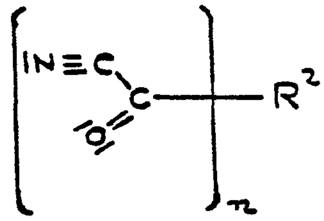 III

sous l'action de HHal, et on libère les produits de réaction par hydrolyse, les symboles Hal, n, $R^1$ et $R^2$ ayant les significations indiquées dans la revendication 1.